Europäisches Patentamt

European Patent Office

Office européen des brevets

(19)

(11) **EP 1 040 799 A1**

(12) **EUROPEAN PATENT APPLICATION**

(43) Date of publication:
**04.10.2000  Bulletin 2000/40**

(51) Int Cl.$^7$: **A61F 13/15**

(21) Application number: **99105191.3**

(22) Date of filing: **01.04.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **THE PROCTER & GAMBLE COMPANY**
**Cincinnati, Ohio 45202 (US)**

(72) Inventors:
• **Carlucci, Giovanni**
  **66100 Chieti (IT)**

• **Veglio, Paolo**
  **65125 Pescara (IT)**
• **Di Cintio, Achille**
  **65126 Pescara (IT)**
• **Bonelli, Guido**
  **65129 Pescara (IT)**

(74) Representative: **Hirsch, Uwe Thomas M.H.**
**Procter & Gamble European Service GmbH**
**65823 Schwalbach am Taunus (DE)**

(54) **Absorbent article with improved breathable backsheet comprising reduced basis weight nonwoven**

(57)    The present invention relates to absorbent articles in particular sanitary napkins with improved comfort, particularly by the use of breathable backsheets whilst maintaining protection level performance. In particular this has been achieved by the use of an improved breathable backsheet comprising at least a first breathable layer and at least a second breathable layer of a fibrous nonwoven web having a basis weight of less than 40 g/m2.

EP 1 040 799 A1

**Description**

Field of the invention

The present invention relates to absorbent articles in particular to sanitary napkins with improved comfort. According to the present invention the articles are provided with breathable backsheet. Preferably, the breathable backsheet comprises at least a first and a second breathable layer.

Background of the Invention

The primary consumer needs which underlie development in the absorbent article field, in particular sanitary napkins, catamenials, or pantiliners is the provision of products providing both a high protection and comfort level.

One means for providing consumer comfort benefits in absorbent articles is by the provision of breathable products. Breathability has typically concentrated on the incorporation of so called 'breathable backsheets' in the absorbent articles. Commonly utilized breathable backsheets are microporous films and apertured formed films having directional fluid transfer as disclosed in for example US 4 591 523. Both these types of breathable backsheets are vapor permeable allowing gaseous exchange with the environment. This thereby allows for the evaporation of a portion of the fluid stored in the core and increases the circulation of air within the absorbent article. The latter is particularly beneficial as it reduces the sticky and soiled feeling experienced by wearers of or associated with articles comprising an apertured formed film or film like topsheet.

The use of apertured film topsheets has long been recognized as providing particular benefits in controlling the liquid flow through this layer into an absorbent structure and reducing the liquid flow out of the absorbent structure towards the skin of a wearer. In this respect apertured film topsheets have provided an exceptional dryness comfort to the wearers of absorbent articles, particularly sanitary napkins. This comfort benefit, however, started to wear off under stress conditions of such articles, such as physical exercising of the wearer (which also caused transpiration from the skin opposite the article to emanate more strongly), heavy loading of the article or extended wearing duration.

A drawback associated with the use of breathable backsheets in absorbent articles is the negative effect on the protection level performance by leakage, known as wet through, onto the users garment. Although, breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the product is utilized during physical exertion, or for heavy discharge loads or over extended periods of time. Thus, whilst the incorporation of breathable backsheets in absorbent articles is highly desirable from a comfort standpoint, since the primary role of a backsheet still remains the prevention of liquid leakage, conventional breathable backsheets have not been successfully incorporated into products.

The problem of wet through onto users garments due to the incorporation of such breathable backsheets in absorbent articles has indeed also been recognized in the art. Attempts to solve the problem have mainly resided in the use of multiple layer backsheets such as those illustrated in US 4 31 216. Similarly European patent application no. 710 471 discloses a breathable backsheet comprising an outer layer of a gas permeable, hydrophobic, polymeric fibrous fabric and an inner layer comprising an apertured formed film having directional fluid transport. The backsheet construction preferably has no liquid transport/wet through under certain specified test conditions. Also European patent application no. 710 472 discloses a breathable backsheet consisting of at least two breathable layers which are unattached to one another over the core area. The backsheet construction preferably has no liquid transport/wet through under certain specified test conditions.

US 4 713 068 discloses a breathable clothlike barrier for use as an outer cover for absorbent articles. The barrier comprises at least 2 layers, a first layer having a specified basis weight, fiber diameter and pore size and a second layer comprising a continuous film of poly (vinyl alcohol) having a specified thickness. The barrier also has a specified water vapor transmission rate and level of impermeability.

However, these proposed solutions have not been able to provide a fully satisfactory solution to the problem of breathable backsheet wet through under stress conditions. But especially under such stress conditions breathability would have most pronounced comfort benefits especially for articles comprising film topsheets. For such articles the experienced or assumed stickiness, stuffiness, or soil residue between film and skin is greatest under stress conditions.

US 5,591,510 as well as WO 97/03118 and WO 97/03795 disclose an apertured film layer having capillaries which are disposed at an angle relative to the plane of the film, which films are referred to as slanted capillary films. This film structure is provided as a improvement for incorporation into clothing and garments which are breathable, yet non transmitting liquids toward the wearer of such garments. European Patent Application No. 98101867.4 filed on February 4, 1998 and European Application No. 98101868.2 filed on February 4, 1998 disclose the use of such slanted capillary films as a layer in breathable backsheets for particularly selected disposable absorbent article designs, particularly sanitary napkins.

EP-A-0813849 provides breathable absorbent articles whose individual elements must meet certain key functional parameter criteria in terms of comfort provision and/or protection such as the backsheet wet through/liquid permeability, the topsheet dryness, the core caliper and core vapor or vapor/air permeability. Furthermore, these elements are combined such that the resultant product, in addition to these individual elements, meets overall criteria such that it has a certain dryness index and sensory index. EP-A-0813849 identifies the key components which affect the principle comfort requirements of flexibility, breathability, dryness and caliper and the key components of protection such as liquid retention/wet through and rewet. The specific combination of these components provides an article delivering both high protection levels as well as high comfort to the consumer.

While the above mentioned EP-A-0813849, which is incorporated herein, gives general criteria for making an effective breathable absorbent product, particularly a sanitary napkin, and also gives specific examples of sanitary napkins according to the invention, a need for an improved breathable absorbent product still remains, particularly referring to an absorbent disposable product having a further improved comfort and a cheaper cost.

It is therefore an objective of the present invention to provide a disposable absorbent article having improved comfort and being substantially cheaper than those products disclosed in the above mentioned European Patent Application.

Summary of the Invention

The present invention relates to a breathable disposable absorbent article comprising the following elements: a liquid pervious topsheet, an absorbent core and a breathable backsheet, said absorbent core being positioned intermediate said topsheet and said backsheet.

The topsheet has a liquid retention of less than 0.22g for a 2.0g load in a topsheet liquid retention test defined below, the absorbent core has a caliper of less than 12mm and has a vapor permeability of at least 200g/m$^2$/24hrs. as defined in the vapor permeability test below, and the breathable backsheet has a liquid permeability of less than 0.16g for a 15ml. load as defined in the liquid permeability test below. According to the invention the topsheet, the absorbent core, and the breathable backsheet are joined such that said breathable at least a first and a second absorbent article has a dryness index of greater than 0.5 and a sensory index of greater than 300.

Furthermore, the article according to the invention has a Modified Sensory Index of at least 200 as defined hereinafter. The backsheet comprises at least a first and a second breathable layer.

Preferably, the first layer is a resilient, three dimensional web which consists of a liquid impervious polymeric film having apertures forming capillaries. The second breathable layer is a porous web which consists of a synthetic fibrous nonwoven web having a basis weight of less than 40 g/m2.

Most preferably, the backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer of a porous web which is a fibrous nonwoven composite web of a meltblown nonwoven layer made from synthetic fibers having a basis weight of less than 13 g/m2 and of a spunbonded nonwoven layer made from synthetic fibers.

Detailed Description of the Invention

The present invention relates to absorbent disposable articles such as sanitary napkins, panty liners, incontinence products, sweat pads, breast pads and baby diapers. Typically such products comprise the elements of a liquid pervious topsheet, a backsheet and an absorbent core intermediate said topsheet and said backsheet. According to the present invention the topsheet, backsheet and core may be selected from any of the known types of these components provided that they meet certain comfort and protection performance requirements detailed herein. Furthermore, the individual elements are joined, preferably utilizing optimized joining techniques such, that the final product also meets specific comfort and performance level criteria also described herein.

Absorbent article components

The topsheet

According to the present invention the absorbent article comprises as an essential component a topsheet. The topsheets suitable for use herein may be any topsheet known in the art provided it satisfies the liquid retention test at 2.0 g load with less than 0.22 g retention. The test details are disclosed below.

The topsheets for use herein may comprise a single layer or a multiplicity of layers. In a preferred embodiment the topsheet comprises a first layer which provides the user facing surface of the topsheet and a second layer between the first layer and the absorbent structure/core. The topsheet typically extends across the whole of the absorbent structure and can extend into and form part of or all of the preferred sideflaps, side wrapping elements or wings.

The topsheet as a whole and hence each layer individually needs to be compliant, soft feeling, and non-irritating to the wearer's skin. It also can have elastic characteristics allowing it to be stretched in one or two directions. As used herein the topsheet hence refers to any layer or combination of layers whose principle function is the acquisition and transport of fluid from the wearer towards the absorbent core.

According to the present invention the topsheet may be formed from any of the materials available for this purpose and known in the art, such as non wovens fabrics, films or combinations of both. In a preferred embodiment of the present invention at least one of the layers of the topsheet comprises a liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523. However, even non-woven or woven substrates can be apertured to improve their function of liquid acquisition.

Backsheet

The absorbent article according to the present invention also comprises a breathable backsheet. The backsheet primarily prevents the extrudes absorbed and contained in the absorbent structure from wetting articles that contact the absorbent product such as underpants, pants, pyjamas and undergarments thereby acting as a barrier to fluid transport. According to the present invention the breathable backsheet has a liquid permeation of less than 0.16 g for a 15 ml load according to the liquid permeation test disclosed in detail below. In addition however, the breathable backsheet of the present invention preferably permits the transfer of water vapor and air through it and thus allows the circulation of gases into and out of the backsheet. The backsheet typically extends across the whole of the absorbent structure and can extend into and form part of or all of the side flaps, side wrapping elements or wings.

According to the present invention a dual or multiple layer breathable backsheet composite is used in the absorbent article. According to the present invention suitable breathable backsheets for use herein comprise at least a first and a second layer. Preferred breathable backsheets for use herein are those having both a high vapor and high air exchange.

The first layer is positioned between the garment facing surface of the absorbent core and the wearer facing surface of the second layer. It is oriented such that it retards or prevents liquid from passing from the absorbent core towards the outside while allowing free air flow and water vapor through it.

According to the present invention also the second layer provides water vapor and air permeability so as to support breathability of the article. In addition to water vapor permeability the air permeability is desirable in order to further improve the comfort benefit from the breathability of the article. In this context suitable water vapor and air permeable layers preferably include fibrous nonwoven webs, or wovens.

The first layer according to the present invention is preferably in direct contact with the absorbent core. It provides air and water vapor permeability by being apertured. Preferably this layer is made in accordance with the aforementioned US-A-5,591,510 or PCT WO-97/03818, WO-97/03795. In particular, this layer comprises a polymeric film having capillaries . The capillaries extend away from the wearer facing surface of film at an angle which is less then 90 degrees. Preferably the capillaries are evenly distributed across the entire surface of the layer, and are all identical. However, layers having only certain regions of the surface provided with apertures, for example only an area outside the region aligned with the central loading zone of the absorbent core, maybe provided with capillaries according to the present invention.

Methods for making such three-dimensional polymeric films with capillary apertures are identical or similar to those found in the apertured film topsheet references, the apertured formed film references and the micro-/macroscopically expended film references cited above. Typically a polymeric film such as a polyethylene (LDPE, LLDPE, MDPE, HDPE or laminates thereof) or preferably a monolithic polymeric film is heated close to its melting point and exposed through a forming screen to a suction force which pulls those areas exposed to the force into the forming apertures which are shaped such that the film is formed into that shape and, when the suction force is high enough, the film breaks at its end thereby forming an aperture through the film.

Especially using a monolithic polymer film as the material for the first layer provides water vapor permeability even under stress conditions. While the apertures provide air permeability during "leakage safe" situations but close the capillaries under stress conditions the monolithic material maintains water vapor permeability in such a case. Preferred breathable monolithic film materials for use herein are those having a high vapor exchange. Suitable monolithic films include Hytrel (TM), available from DuPont Corporation, USA, and other such materials as described in Index 93 Congress, Session 7A "Adding value to Nonwovens", J-C. Cardinal and Y. Trouilhet, DuPont de Nemours international S.A, Switzerland.

Various forms, shapes, sizes and configurations of the capillaries are disclosed in EPA 98101867.4 filed on February 4, 1998 and EPA 98101868.2 filed on February 4, 1998 both of which are herein incorporated for reference.

In particular the apertures form capillaries which have side walls . The capillaries extend away from the wearer facing surface of the film for a length which typically should be at least in the order of magnitude of the largest diameter of the aperture while this distance can reach up to several times the largest aperture diameter. The capillaries have a first opening in the plane of the garment facing surface of the film and a second opening which is the opening formed when the suction force (such as a vacuum) in the above mentioned process creates the aperture. Naturally the edge of the second opening may be rugged or uneven, comprising loose elements extending from the edge of the opening. However, it is preferred that the opening be as smooth as possible so as not to create a liquid transport entanglement between the extending elements at the end of the second opening of the capillary with the absorbent core in the absorbent article (in contrast this may be desirable for apertured film topsheets where such loose elements provide the function of sucker feet). The capillaries in the first layer of the breathable backsheet allow air and water vapor permeability which is not hindered by them being slanted at an angle or by the shape. At the same time the slanting and shaping will allow the capillaries to close under pressure excerpted from the wearer facing side on them such that liquid transport through the capillaries towards the outside of the article becomes nearly impossible. Hence these three-dimensional formed film layers are highly preferable in the context of breathable absorbent articles and in particular so with the additional second outer layer which is provided as hereinafter explained.

The second outer layer of the breathable backsheet according to the present invention is a fibrous nonwoven web having a basis weight of less than 40 g/m2, preferably of less than 28 g/m2. More preferably, the second outer layer is a fibrous nonwoven web formed by a layered composite of a meltblown nonwoven layer made from synthetic fibers having a basis weight of less than 13 g/m2 and of a spunbonded nonwoven layer also made from synthetic fibers.

It has been discovered that contrary to example 2a of EP-A-0813849, an absorbent product, particularly a breathable sanitary napkin, having a backsheet comprising at least one breathable layer of a resilient, three dimensional web which consists of a liquid impervious polymeric film having apertures forming capillaries which are not perpendicular to the plane of the film but are disposed at an angle of less than 90° relative to the plane of the film, and at least another breathable layer of a porous web which consists of a fibrous nonwoven web having a basis weight of less than 40 g/m2 (particularly of about 28 g/m2), functions very well in term of comfort, soiling of the user panty, dryness, etc. while providing additional comfort due to the reduced basis weight of the non-woven layer. This reduction of basis weight also provides an improved material consumption structure of the whole article.

Absorbent core

According to the present invention the absorbent cores suitable for use herein may be selected from any of the absorbent cores or core system known in the art provided that certain requirements as concerns caliper and vapor and/or air permeability as defined in EP-A- 0813849 are meet. As used herein the term absorbent core refers to any material or multiple material layers whose primary function is to absorb, store and distribute fluid. In particular, the absorbent core of the present invention should have a high vapor permeability preferably also a high air permeability. The absorbent core according to this invention has a caliper or thickness of less than 12mm, preferably less than 8mm, more preferably less than 5mm, most preferably from 4mm to 2mm.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralized, polymeric gelling material. This material forms a hydrogel upon contact with water Such polymer materials can be prepared from polymerizable, unsaturated, acid-containing monomers which are well known in the art.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents.

Absorbent article construction

A further aspect of the present invention relates to the joining of the topsheet, backsheet and absorbent core elements to provide the absorbent article. According to the present invention at least two, preferably all of the elements of the article are joined.

Each of said elements comprising at least one layer has a wearer facing surface and a garment facing surface. Typically, adjacent garment facing surfaces form a common interface with the wearer facing surface of an adjacent element or layer. The elements or layers are joined together across this common interface. In this manner the topsheet is joined to the absorbent core, and the core is joined to the backsheet. Furthermore, each of said topsheet, backsheet and core elements may comprise more than one layer and these layers may also be similarly joined. In addition the topsheet may be directly or indirectly by joined to the backsheet at the periphery of the absorbent article.

The elements and layers thereof may be joined by any means known in the art for affixing two adjacent layers of material, such that the layers are directly attached to one another or directly attached to one another via the joining means. Suitable joining means include adhesive, fusion bonding, ultra sonic bonding, stitching, heat (e.g. thermobonding by welding fibers at intersections or melting a polymer to attach fibers or films to each other), crimping, embossing, and/or pressure bonds, or dynamic mechanical bonds. According to an embodiment of the present invention the preferred means of joining is adhesive. Suitable adhesives include non pressure sensitive and cold adhesives. The adhesive may be applied by any means known in the art such as application in form of lines, spiral application, slot coating, spraying, spiral spraying, curtain coating, control coating and printing, provided that the adhesive does not substantially affect the breathability and other functions of the elements of the article.

In a preferred embodiment of the present invention the inter element or inter layer joining adhesive is selected and applied so as to reduce any impact it may have on the breathability of the absorbent article and preferably also the flexibility of the absorbent article. Since many commonly utilized adhesives are not vapor permeable it is highly preferable to minimize the amount of adhesive used to join the layers/ elements of the absorbent article in order to minimize their impact on the permeability (breathability)and preferably also the flexibility of the absorbent article. One means of achieving this is to use particular adhesive application methods such as open adhesive application techniques, whereby areas of the common interface are adhesive free, whilst retaining the required level of attachment/ joining of the two adjacent layers or elements. In particular, open adhesive patterns, which are substantially neutral to the flexibility of the article, such as a light spiral spraying or an application in form of spaced fine lines are preferred. The layers and elements should be joined in such a manner so that the absorbent article product maintains structural integrity but no more. This method and particularly the application of few fine lines of adhesive finds particular application for the interlayer joining of the backsheet element layers and the joining of the backsheet element and the absorbent core element. Alternatively adhesives which are vapor permeable may be used.

Preferably not more than 40%, more preferably less than 20%, most preferably less than 10% of the common interface of two adjacent layers or elements is joined. Furthermore, the density of the adhesive should be reduced and a thin application of adhesive is preferred.

In a preferred embodiment of the present invention wherein the absorbent article finds utility as a sanitary napkin or panty liner, the absorbent article is also provided with a panty fastening means which provides means to attach the article to an undergarment. For example the panty fastening means may comprise a mechanical fastener such as hook and loop fasteners such as marketed under the tradename VELCRO, snaps or holders. Alternatively, the article is fastened to the undergarment by means of panty fastening adhesive on the backsheet. The panty fastening adhesive provides a means for securing the article to the panty and preferably a means for securing the article when soiled, to the fold and wrap package for convenient disposal. Typically, at least a portion of the garment facing surface of the backsheet is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

In order to reduce the effect of the breathablility of the backsheet and thus of the article as a whole, the adhesive is preferably applied such that at least 60%, preferably from at least 80%, most preferably at least 90% of the garment facing surface of the backsheet is adhesive free.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX EI/O and BL 30 MG-A SILOX 4 P/O available from Akrosil Corporation.

According to the present invention the absorbent article can be used beneficially in the context of sanitary napkins, panty liners, incontinence articles and diapers. However, sanitary napkins are particularly susceptible to the present invention. The disposable article may thus also have all those features and parts which are typical for products in the context of their intended use.

## DEFINITIONS OF INDEX AND TEST METHODS

According to the present invention the absorbent elements meeting the requirements as described herein above must as an essential requirement be combined such that the resultant absorbent article product meets certain performance and comfort indexes herein referred to as the sensory index, the modified sensory index and the dryness index. The dryness index is a function of the effective breathability test and rewet test of the absorbent article and the sensory indexes are a function of the effective breathability test, flexibility, edge flexibility, and caliper of the absorbent article. The test methods are defined hereinafter. The indices are defined by the equations below:

$$\text{Dryness index} = \text{Effective breathability} / (\text{Rewet test value})$$

$$\text{Sensory index} = \text{Effective breathability} / (\text{Flexibility x Caliper})$$

$$\text{Modified Sensory Index} = \text{Effective Breathability} / (\text{Edge Flexibility x Caliper})$$

Dryness Index

The Dryness Index is a reflection of one of the unexpected interactions that an absorbent article, particularly a sanitary napkin needs to satisfy, in order to provide overall dryness and/or comfort benefits to the wearer of the product. As such the dryness index reflects the perceived wetness during use. It is determined by both the dryness of the wearer facing surface of the product that lies closest to the body in use (i.e. absorbent article rewet test) and the dryness that can be achieved via water vapor exchange with the environment and air circulation via the backsheet (i. e. effective breathability).

Effective Breathability:

The effective breathability is empirically determined from the equation below:

$$\text{Effective Breathability} = \text{Vapor Permeability} + 0.25 \text{ x Air Permeability}$$

The effective breathability determines a numerical value for the breathability. It considers the two key mechanisms that participate in exchange of humidity and temperature while wearing a absorbent article having a breathable backsheet. The first mechanism is water vapor exchange via the process of diffusion. This is a continuous process and the mechanism is well understood and represented by a simple diffusion equation. In addition, body motion can result in a change in the relative position of the wearers body and the absorbent article, for example between a sanitary article and the body known as gapping. This motion also is accompanied by a process of air exchange. Repetitive bodily motion can quite literally pump air into and out of the backsheet or at the sides of the product, where a product may not maintain intimate contact to the body. Naturally the stiffer an absorbent article is in the genital region the less likely is this process of pumping to deliver an additional benefit to simple vapor exchange since the product is less deformable and is likely to press up closer to the body like a gasket.

Sensory Index

The Sensory Index is an index that quantifies the relationship between product attributes that need to be satisfied in addition to breathability to deliver a true benefit in use. This is due the interactions between breathability, the product caliper and product stiffness/flexibility.
Thus in short, the sensory index value gives an indication of the range of values of the breathability, flexibility and caliper of the product according to the present invention which provides protection and comfort benefits.

Modified Sensory Index

The Modified Sensory Index is also an index that quantifies the relationship between product attributes that need to be satisfied in addition to breathability to deliver a true benefit in use. This is due the interactions between breathability, caliper, flexibility at the edge of the product (particularly at the edge of the product absorbent core).
Thus in short, the modified sensory index value gives an indication of the range of values of the permeability, edge flexibility (particularly at the edge of the absorbent core product), and caliper of the product according to the present invention which provides an improvement to protection and comfort beyond the prior art, particularly beyond EP-A-0813849.

**Absorbent article component tests**

**Topsheet Retention test:**

The topsheet retention test is utilized to assess the liquid retention character (of bodily discharges) of topsheet materials or composites that may be used on disposable absorbent articles and particularly sanitary napkins.

**Basic Principle of the Methods:**

The basic principal of the test is to evaluate the liquid retention behavior of alternative topsheet materials to liquids that simulate bodily discharges. A "good topsheet material" in this test can be a film (e.g. apertured formed film) or a fibrous or a fibrous nature film provided it has a low propensity to acquiring and retaining liquids either on or within its structure. Naturally a "good topsheet" is also, in addition to having low retention properties expected to allow rapid transmission of bodily discharges further into the article and to hinder discharges that are contained within the article from returning to the upper (body side) surface of the article. Additionally a "good topsheet" should also maintains a clean appearance during use of the articles.

To assess the topsheets retention to liquid, a test is performed as detailed below:

Two sheets (with dimensions of 5 cm x 5 cm) of a commercially available airlayed absorbent tissue each with a basis weight of 63 g/m$^2$ available from Walkisoft USA under the supplier code Metmar (P50W.IPED) are utilized to simulate an absorbent core.

A sample of the topsheet material (with dimensions of 5 cm x 5 cm) that is to be assessed is placed directly on top of this absorbent structure. A standardized test liquid closely matching menses in viscosity and electrical conductivity (see below) is dripped onto the center of the test sample from a height of 3 cm and at a rate of 2 g/min. until a total of 2 grams has been introduced onto the sample. The sample is left without further interference for a period of 1 minute.

Following the 1 min. waiting period a perspex block (1 cm thick with dimensions 8.5 cm x 8.5 cm) is placed on top of the test sample and a weight is lower gently onto the total assemble for a period of 5 minutes. The total pressure exerted onto the test sample, at this point, is 70 g/cm$^2$.

The weight and perspex block is removed and carefully the topsheet sample is removed and placed on a stack of 2 sheets (with dimensions 12 cm x 12 cm) of commercially available filter/blotting paper {produced by Cartiera Favini S.p.A. Italy; Type Abssorbente Bianca "N30" (local vendor Ditta Bragiola SpA. Perugia, Italy)} that have been pre-weighed. A second stack of 2 pre-weighed filter papers are placed on top of the topsheet sample. A second weight is placed on top of the filter paper stack containing the topsheet sample. The second weight exerts a pressure onto the filter paper stack of 130 g/cm$^2$ for a period of 15 seconds. The weight (for this critical step) is attached to a hydraulic arm. The lowering of the weight and time the sample is placed under pressure is controlled via a simple electronic device to ensure reproducibility from one test to the next.

The second weight is removed and each (lying above and below the topsheet sample) filter paper stack is weighed and the difference for each surface (liquid pick-up from the topsheet sample) recorded. Materials that have a zero pick-up value for the top surface (placed normally in contact with the users skin) are given a zero *"topsheet retention"* value independent of having a non-zero value for the bottom surface as these materials clearly demonstrate non-communication between the upper and lower surfaces.

**Test Solution:** Preparation of Test Solution Paper Industry Fluid (PIF)

The test solution PIF is a widely used test liquid in the Paper industry due to its simple composition, ability to prepare and maintain high standards of solution quality and its similarity to human menses with respect to viscosity and ionic surface tension.

The solution PIF is prepared by dissolving the following reagent components, at the indicated quantities, into 1 liter of distilled water. Care should be taken in dissolving the solid components and particularly the Carboxylmethyl-cellulose. Typically the solid components should be added over a period of one hour slowly and with constant stirring of the solution (via a magnetic stirring device).

Supplier Sigma Chemicals, USA

| Chemical Component - | | Usage / 1L |
|---|---|---|
| 1) | Carboxymethylcellulose, Sodium salt low viscosity: Order No. = C 5678, | 15 grams |
| 2) | Sodium bi-Carbonate, Crystalline: Order No. = S 8875 | 4 grams |

(continued)

| Chemical Component - | | Usage / 1L |
|---|---|---|
| 3) | Sodium Chloride (AR): Order No. = S 9625 | 10 grams |
| 4) | Glycerol (>99% pure): Order No. = G 5516 | 80 grams |

**Vapor and Air Permeability test: Absorbent core**

The air permeability test is utilized to assess the ability of the absorbent core to exchange/circulate vapor and preferably air and is carried out on the core material as detailed for an absorbent article.

**Liquid Permeability Test: Backsheet**

The liquid permeability test is utilized to quantify the barrier properties of breathable backsheet materials or constructions that could be utilized on a breathable absorbent article and particularly on a sanitary napkin.

**Basic Principle of the Methods:**

The basic principle of the test is to evaluate the performance of backsheet materials or constructions to liquids which simulate bodily discharges. A "good backsheet layer or construction" is expected to be sufficiently open to be classified as breathable but, without being too open to the passage of bodily discharges. To ensure that this test is sufficiently representative to the situation when the absorbent article is actually used a test solution closely resembling human menses is utilized, referred to herein as Artificial Menstrual Fluid (AMF). AMF is based on modified sheeps' blood as detailed in the solution preparation method detailed below.

To determine the liquid permeability of a backsheet or backsheet construction, a standard absorbent structure with the backsheet material or construction is prepared and placed flat on a see through test stand made of perspex. The sample to be tested is oriented with the absorbent structure exposed (upper side) and the breathable backsheet side in contact with the perspex test stand (bottom side). Suspended above the sample to be analyzed is a liquid delivery system that is capable of delivering any desired quantity of the test liquid.

The standard absorbent structure is composed of 4 layers (folded as a stack) of airlayed tissue of 63 g/m$^2$ basis weight {available from Walkisoft USA under the supplier code Metmar (P50W.IPED)} having dimensions of 20 cm x 6.5 cm. The backsheet is then placed on top of this structure without any additional adhesive attachment.

Located between the back most surface of the test sample and the see through test stand are two sheets of absorbent filter paper {produced by Cartiera Favini S.p.A. Italy; Type Abssorbente Bianca "N30" (local vendor Ditta Bragiola SpA. Perugia, Italy)}. The absorbent filter paper is in intimate contact with the backsheet of the test sample to simulate, for example a sanitary napkin attached to a panty or a diaper/incontinence device in close contact with the clothing. Directly below the see through test stand is a mirror so positioned to allow any red color change in the absorbent filter paper to be continuously observed. For example, if the backsheet is unable to adequately resist liquid transmission then the filter paper will become wet with the red AMF solution and this can be observed in the mirror. The magnitude of the transmitted solution is determined by simply weighing the absorbent filter paper.

The test solution is introduced to the test sample via a calibrated delivery system such as via a simple burette according to the desired test approach as detailed below. The loading of the pad follows in a step wise manner as typically occurs in-use. Once loaded the test sample is then placed under a pressure of 70 g/cm$^2$ which is believed to reflect more stressful pressures that are nevertheless regularly obtained in-use. The test sample remains under the 70 g/m$^2$ pressure for a period of up to 5 mins. At which time the weight is removed and the absorbent filter paper is weighted to determine if and to quantify the extent of liquid that has been transported through the backsheet or backsheet construction.

The process is then repeated entirely with an additional introduction of liquid to the test sample. For each introduction of liquid a new stack of absorbent filter papers (pre-weighted) is used to be able to better determine the liquid barrier behavior as a function of the load.

The loading steps are specifically:

Step 1          5 ml
Step 2          1 ml
Step 3 - 11     1 ml additions each step to a total loaded volume of 15 ml.

A good backsheet layer or layered construction is expected to have zero wet-through (absorbent filter paper remains clean and white and does not change weight) throughout the entire stepwise loading program (i.e. until 15 ml load).

**Preparation of Test Liquid AMF**

Artificial Menstrual Fluid (AMF) is based on modified sheep's blood that has been modified to ensure it closely resembles human menstrual fluid in viscosity, electrical conductivity, surface tension and appearance. In addition we introduce a surfactant (1%) to this test fluid (supplied by by Pegesis/USA) to better reflect stress situations in which typical hygiene practice (and in some limited situations, dietary influences) may introduce additional surfactants or unexpected levels of, for example, fatty acids, that might lower the blood surface tension. Low surface tension menses is the biggest contributor to through backsheet wet-through failure on a breathable absorbent article such as a sanitary article.

**Reagents:**

1) Difibrinated sheep's blood is available from Unipath S.p.A {Garbagnate Milanese/Italy}.
2) Lactic Acid from J.T. Baker Holland Reagent Grade (85-95%w/w)
3) Potassium Hydroxide (KOH) from Sigma Chemical Co. USA, Reagent grade
4) Phosphate Buffer Saline Tablets from Sigma Chemical Co. USA, Reagent grade
5) Sodium Chloride from Sigma Chemical Co. USA, Reagent grade
6) Gastric Mucine from Sigma Chemical Co. USA,Type III (CAS 84082-64-4)
7) Distilled Water.

Step 1:
Prepare a $9 \pm 1$ % Lactic Acid Solution by dissolution of lactic acid powder and distilled water.
Step 2:
Prepare a 10% Potassium Hydroxide (KOH) solution by dissolving KOH powder into distilled water.
Step 3:
Prepare a Phosphate buffer solution buffered to pH = 7.2. by dissolving tablets as directed into 1 L distilled water.
Step 4:
Prepare and slowly heat to $45 \pm 5$ °C a solution of the following composition:

- $460 \pm 5$ ml of phosphate buffer solution
- $7.5 \pm 0.5$ ml of KOH solution

Step 5:
Prepare a Mucous Solution by slowly dissolution (with constant stirring) of approximately 30 grams of gastric mucine in the pre-heated ($45 \pm 5$ °C) solution prepared in step 4. Once dissolved the solution temperature should be increased to between 50 - 80 °C and the mixture covered for approximately 15 mins. Turn the heat down to maintain a relatively constant temperature between 40 and 50 °C and continue to stir for a period of 2.5 hrs.
Step 6:
Remove the solution from the hot plate and allow the solution (from step 5) to now cool to less than 40 °C. Add 2.0 ml of the 10% lactic acid solution and mix thoroughly for 2 mins.
Step 7:
Place the solution in an Autoclave and heat to a temperature of 121 °C for 15 mins.
Step 8:
Allow the solution to cool to room temperature and dilute 1 to 1 with the difibrinated sheep's blood.

Following AMF preparation its viscosity, pH and conductivity are measured to ensure the blood characteristics lie in a range close to that of normal menstrual blood {(see reference H.J. Bussing "zur Biochemie de Menstrualblutes" Zbl Gynaec, 179,456 (1957)}. The viscosity should lie in the range of 7 to 8 (units cStK). The pH should lie in the range of 6.9 to 7.5 and the conductivity in the range 10.5 to 13 (units mmho). If the viscosity is not within the range specified above it should not be used and a new batch of AMF needs to be prepared. This may require adjustment to the quantity of gastric mucine used. Since this is a natural product its composition may alter from one lot to another.
For individual measurements typically 100 ml AMF test solution with surfactant is prepared by mixing 90 ml AMF solution (maintained at 25 °C) with 10 ml Surfactant. The AMF/1% surfactant solution must be constantly mixed to ensure the components do not separate prior to usage. The solution should be used only within 4 hours of preparation.

**Absorbent article tests**

The following tests were carried out on selected exemplified absorbent articles detailed below:

**Air & Vapor Permeability Test on absorbent article products**

The Vapor permeability test is utilized to quantify the vapor transmission properties of breathable absorbent articles.

**Basic Principle of the Methods:**

The basic principle of the test is to quantify the extent of water vapor transmission of an absorbent article. The test method that is applied is a standard one, namely ASTM E 96 - 80, Procedure B - Water Method at 23°C.. The test is performed in a stable temperature/humidity laboratory maintained at a temperature of 23° C at 50% RH for a period of 24 hours.
The vapor permeability value is determined as the weight loss divided by the open area of the sample holder and quoted per day.

$$\text{i.e. Vapor Permeability} = \text{Weight Loss (g)} / (0.003 \text{ m}^2 / 24 \text{ hrs})$$

**Air Permeability test:**

The air permeability test is utilized to assess the ability of an absorbent structure to circulation/exchange air.

**Basic Principle of the Methods:**

The basic principle of the test is to evaluate the resistance of an absorbent article to the passage of air. In this test, the volume (or amount) of air that flows through an article of given dimensions under standard conditions (of 23 °C /50% RH) is measured. The instrument utilized for the test is: Air Permeabilimeter FX 3300 manufactured by TexTest AG Switzerland.
Samples should be allowed to equilibrate in the test environment for at least 4 hrs prior to commencement of the measurement. The article (having dimensions exceeding 5 cm$^2$ the dimensions of the measurement head) is placed on the device as instructed by the manufacturer. An aspiration pump set to generate a pressure of 1210 kPa that sucks air through the sample layer or structure. The device measures the volume of air flow and the pressure drop across the orifices that contains the sample and measurement head. Finally the device generates a value of air permeability in the units of liters/m2/s.

**Rewet Test:**

The rewet test applied here is standard in the absorbent article field. The rewet test method gives an indication of the amount of liquid expelled under pressure through the topsheet.

**Basic Principle of the Methods:**

In this context the rewet method is utilized to assess the dryness of the product with respect to the wearer facing surface of the product . In combination with additional test methods that assess the dryness of the product with respect to the openness of the backsheet (air & vapor permeability test) it allows the total product dryness to be represented.
The test solution that is utilized for this test is based on the Paper Industry Fluid (PIF) that has been detailed above.

**Apparatus:**

1) Blotting Paper available from Schleicher & Schuell (Germany). S & S Rundfilter/Durchmesser 150 mm, No. : 597, Reference-No.: 311812.
2) A weight of 4200 g covered on the lower surface with a foam of moderate flexibility. Both the weight and foam are covered with a thin, flexible plastic film to maintain waterproofs. The weight dimensions should allow a 6 cm x 10 cm surface to contact the article under examination. Pressure exerted onto the article = 70 g/cm$^2$.
3) A perspex (7 mm thick) plate of dimensions 6 cm x 10 cm with a hole of dimensions 3 cm x 4 cm centred in the template.
4) A burette capable of introducing the test fluid at a reproducible rate of 7 ml in 90 seconds.
5) An analytical balance capable of reading to 4 decimal places.

**Sample Preparation / measurement:**

The article to be assessed is removed from a packaging and placed on a flat laboratory surface and centered directly below the burette for test liquid delivery. The perspex plate is placed on the surface and the PIF test liquid is introduced over the exposed area corresponding to the hole in the perspex plate. After 90 seconds 7 ml of PIF have been introduced to the sample and an electronic counter set to 20 min's is activated. During this waiting period a stack of 7 discs of filter paper are weighed on the analytical balance and noted.
After 20 mins. the perspex plate is removed and the stack of filter papers are positioned centrally on the article being assessed and the weight is gently lowered onto the filter paper stack. The article and filter paper stack remain under the pressure exerted by the weight for a period of 15 seconds, after which the weight is carefully removed and the filter paper stack is re-weighted. The difference in weight (to the nearest milligram) is recorded as the rewet value. The tests are repeated for at least 10 samples to ensure adequate accuracy of the measurements.

**Flexibility Test & Caliper Test:**

**Basic Principle of the Methods:**

The flexibility test is utilized to quantify the product flexibility or product stiffness in the cross direction. Most flexibility tests have attempted to establish a product benefit based on a product design change using a range of flexibility test methods to quantify this such as measuring the drape (bend ability) of a product or the combination of stiffness in both length and cross direction. The flexibility test used herein is a dynamic stiffness measurement (force to deform vs. distance deformed) which determines the resistance of a product to deform in the cross direction. The higher the stiffness value the more the product is likely to push against the sensitive skin of the wearers inner thighs and create a sensory negative during various bodily motions.

**Apparatus:**

1) Climatically controlled Lab.

Maintenance of 23 °C and 50 % Relative humidity.

2) Instron Limited. UK Model 6021

Interfaced to a standard IBM with RS 232 interface for data logging.
Data are sent to the IBM computer in the form of distance and force values.
Data is read into a standard Microsoft Excel worksheet for analysis.

Load cell = 10 N
Initial clamp separation = 50 mm
Final clamp separation = 25 mm
Distance sample to be deformed = 25 mm
Compression speed = 100 mm / 1 mins.

3) Caliper Measurement Device: Mitutoyo Instruments (Japan) Model 543 -601 B

The caliper is measured using a precision digital measurement device ($\pm$ 0.02 mm) with a circular measurement foot of 40 mm diameter that exerts a pressure of 6.2 g/cm$^2$ .

**Sample Preparation:**

The tests are performed on the final form of the product identical in all ways and preferably on the same batch of products to be worn or assessed by a consumer.

In the case of a sanitary napkin, or light incontinence device the product is removed from packaging and any release paper that may be used to maintain adhesives used to attached the article to a panty or other piece of clothing is be removed. Exposed glue surfaces (i.e. panty fastening adhesive) are rendered inactive by lightly applying a talcum powder to the adhesive surface.

**Caliper Measurement.**

The Average caliper of the products is first determined. For products that are inherently flat the caliper at representative points (at least 5) of the product is measured to determine an average value. For products of complex shapes, such as relatively thick in the center and relatively thin in the extremities, a smaller measurement foot on the caliper device (maintaining a measurement pressure of 6.2 g/cm$^2$) is used and to ensure at least 10 measurement points are utilized to more accurately determine the average product thickness.

**Flexibility Measurement.**

The product is attached vertically between the clamps of the Instron device. The clamps are so positioned to start the compression (in product cross direction) from a distance of 50 mm. The sample is compressed over a distance of 25 mm to a final clamp separation of 25 mm. The instrument details are given above.

The Instron records the clamp separation (in mm) and the force exerted to achieve this separation and sends this data via an RS232 interface to an IBM computer equipped with Microsoft windows 3.1 and Microsoft Excel version 4.0. The force and distance data are loaded in to the Excel software and the average force measurements over the full 25 mm compression cycle is determined.

The measurements are performed on 10 samples of the same type to ensure a representative stiffness value to be determined for the sample under investigation.

**Edge flexibility measurement.**

The edge flexibility measurement follows the same procedure and uses the same equipment , as already described in connection to flexibility measurement, with the following differences:

-Samples Preparation:

The sample is cut along the product longitudinal center line to create two equal halves using scissors, while avoiding to compress the sample. In addition, any material which projects beyond the longitudinal edge of the absorbent core of the product is removed by trimming it using scissors, while avoiding to compress the sample.

-Test operation:

The above product sample is attached vertically between the bottom clamp of the Instron device , while the top clamp is replaced by a flat circular steel plate having the following dimensions: diameter 38mm, thickness 18mm. The flat plate has on the bottom surface a folding guide (length 10 mm; height 5mm) to ensure the sample is maintained on the vertical axis of the compression and starts to deform it in a controlled manner.

The bottom surface of the flat plat is in contact with the above mentioned longitudinal absorbent core edge. The bottom clamp and the flat plate are so positioned to start the compression (in product cross direction) from a distance of 25 mm. The sample is compressed over a distance of 16 mm to a final clamp separation of 9 mm.

As in the case of flexibility measurement, the Instron records the separation (in mm) between the bottom clamp and the top flat plate and the force exerted to achieve this separation and sends this data via an RS232 interface to an IBM computer equipped with Microsoft windows 3.1 and Microsoft Excel version 4.0. The force and distance data are loaded in to the Excel software and the average force measurements over the full 16 mm compression cycle is determined.

The measurements are performed on 10 samples of the same type to ensure a representative edge stiffness value to be, determined for the sample under investigation.

Except for the modified sensory index and edge flexibility measurement, the preceding definitions and tests are same or substantially similar to those described in EP-A-0813849.

However it should be remarked that the edge flexibility measurement is more specific to quantify the product flexibility or product stiffness at the longitudinal edges of the product (more specifically at the longitudinal edge of the absorbent core), while the flexibility measurement is more specific to quantify the product flexibility or product stiffness in the cross direction is the central part of the article. Therefore, the edge flexibility test used herein is a dynamic stiffness measurement (force to deform vs. distance deformed) which determines the resistance of a product to deform in the cross direction but also taking into account the product flexibility or product stiffness at the edge of the absorbent core. Consequently, the edge flexibility measurement relates more to a sensory negative during bodily motions due to pushes of product edges against the sensitive skin of the wearers inner thighs.

The value of edge flexibility as well as of flexibility should be less than 1.5 N.

Example

In the following example of the present invention a sanitary napkin incorporating the breathable backsheet according to the invention was made. This napkin, except for the differences hereinafter indicated, is identical to the commercially available sanitary napkin Always Ultra Normal size manufactured , for example, in Germany by Procter & Gamble GmbH. This sanitary napkin is not a breathable one because its backsheet is made of a conventional impervious polyethylene film.

The differences were:

The breathable backsheet is a multi-layer construction composed of two layers. The first layer is placed directly in contact with the absorbent core. It is a formed apertured film, more particularly it is a resilient, three dimensional web which consists of a liquid impervious polymeric film having apertures forming capillaries which are not perpendicular to the plane of the film but are disposed at an angle of less than 90° relative to the plane of the film made of Low Density PE. This film was supplied by Tredegar Film Products B.V. Holland under the manufacturing code TC 21097. This film was joined to the absorbent core by four longitudinal fine lines of hot melt glue. In particular there were two lines of glue on each longitudinal side of the absorbent core: the first one spaced 6 mm inside from edge of the absorbent core at its narrow part (i.e. the central part of the absorbent core which in the present case has a width of about 65 mm as in the above mentioned Always Ultra Normal size) and the second one spaced 12 mm further inside the first one. Each glue line had a width of about 0.8 mm and the glue was an hot melt adhesive supplied under the manufacturing code PM 17 by the Italian firm Savare' I.C. Srl. The first film layer extends at least to the periphery of the absorbent core or a few millimeters beyond.

The bottom most layer that would lie, in-use, directly in contact with the wearer panty (garment facing layer) is composed of a synthetic nonwoven laminate manufactured by Corovin GmbH (BBA Group) in Germany under the manufacturing code V 8/6. The nonwoven laminate is composed of 16 $g/m^2$ basis weight spunbond fiber layer and 11.5 $g/m^2$ basis weight meltblown fiber layer. It is thermosealable and has an hydrophobic treatment.. This second nonwoven backsheet layer is joined to the first backsheet layer by further four fine lines of hot melt glue which are made and positioned in the same manner as above described. The nonwoven laminate was oriented so that the melt blow fiber layer was in contact with the first backsheet layer.

The nonwoven laminate extends beyond the first film layer and absorbent core, to the same extend as the topsheet. The topsheet and the nonwoven laminate were thermosealed by a conventional crimping process all around the perimeter of the sanitary napkin forming a constant width peripheral crimp seal as in the above mentioned sanitary napkin Always Ultra Normal size.

The attachment of the product to the panty is provided by an adhesive supplied by the German firm Fuller GmbH under the manufacturing code Lunatac HL2238X. This hot melt adhesive was applied by coating it directly on the nonwoven laminate ( on its external surface which is made of the spunbond fiber layer) at a basis weight of about 27 g/m2 with the same dimensions of the panty attachment means of the above cited Always Ultra Normal size, i.e. in a rectangular pattern having a width of about 57 mm and a length of about 171 mm.

Contrary to the example 2a of EP-A-0813849, the above described breathable sanitary napkins far exceeded all the numerical requirements of the present invention.

In particular the sanitary napkin of the present invention had the following results according to the test methods indicated herein:

Air permeability: 810 l/m2/s
Vapor permeability: 960 g/m2/24hr
Flexibility: 0.35 N
Caliper: 3.0 mm
Effective Breathability: 1163
Rewet test value: 8 mg
Sensory Index: 1107
Dryness Index: 145
Edge Flexibility: 0.61 N
Modified Sensory Index: 635
Liquid permeability ( of the breathable backsheet ) at 15 ml load: less than 0.16 g.

In comparison the sanitary napkin described in the example 2a of EP-A-0813849 failed in liquid permeability test.

Also it should be appreciated that the fibrous nonwoven web constituting the second backsheet layer of the product according to the present invention has a total basis weight of only 27.5 g/m2, while according to example 2b of EP-A-0 813 849 the total basis weight of the nonwovens constituting the bottom most layer that would lie, in-use, directly in contact with the wearer panty (garment facing layer) had a basis weight of 48 g/m2.

**Claims**

1.  Breathable disposable absorbent article of layered construction, each layer or system of layers having a garment facing surface, which is oriented to face in the direction of a garment during use of the article, and a wearer facing surface, which is oriented to face in the direction of the wearer during use of the article, said article comprising at least:

    -   a liquid pervious topsheet providing the wearer facing surface of said article;
    -   an absorbent core;
    -   a breathable backsheet located on said garment facing surface of said absorbent core, said backsheet comprising at least a first backsheet layer and a second backsheet layer, said first backsheet layer being positioned between said garment facing surface of said absorbent core and said wearer facing surface of said second backsheet, said first and said second backsheet layers being air and water vapor permeable, said second backsheet layer being a fibrous nonwoven web made from synthetic fibers having a basis weight of less than 40 g/m2;

    said topsheet and at least said second backsheet layer have a peripheral non-adhesive attachment therebetween; said second backsheet layer is attached to said first backsheet layer in the region of said absorbent core by an open adhesive pattern which is substantially neutral to the flexibility of the article;

    said topsheet has a liquid retention of less than 0.22g for a 2.0g load in the topsheet liquid retention test,

    said core has a caliper of less than 12mm and has a vapor permeability of at least 200 g/m$^2$/24hrs. as defined in the vapor permeability test, and

    said breathable backsheet has a liquid permeability of less than 0.16g for a 15ml. load as defined in the liquid permeability test;

    said topsheet, said core, and said breathable backsheet are joined to each other such that said breathable absorbent article has a dryness index of greater than 0.5 and a sensory index of greater than 300, and a Modified Sensory Index of greater than 200.

2.  Breathable disposable absorbent article according to claim 1, wherein

    -   said first backsheet layer comprising a resilient, three dimensional web said web consists of a liquid impervious polymeric backsheet film having apertures, said apertures forming capillaries , said capillaries having side walls which extend away from said wearer facing surface of said backsheet film , said capillaries having a first opening in said garment facing surface of said backsheet film and a second opening at the end of said capillaries spaced apart from said wearer facing surface of said backsheet film, said capillaries extend away from said wearer facing surface of said backsheet film at an angle of less than 90° measured from the plane of said backsheet film; and wherein

- said fibrous nonwoven web is a layered composite of a meltblown nonwoven layer and of a spunbonded nonwoven layer, said meltblown nonwoven layer having a basis weight of less than 13 g/m$^2$.

3. A breathable disposable absorbent article according to claim 2 wherein said fibrous nonwoven web has a basis weight of less than 28 g/m$^2$.

4. A breathable disposable absorbent article according to claim 2 or 3 wherein said spunbonded nonwoven layer constitutes the outer garment facing surface of said article.

5. A breathable disposable absorbent article according to any of the preceding claims wherein at least some of said synthetic fibers are hydrophobically treated.

6. A breathable disposable absorbent article according to claim 5 wherein the material from which at lest some of said synthetic fibers are made comprises a fluorocarbon additive which provides an increased hydro-phobicity relative to the same fibers without said fluorocarbon additive.

7. A breathable disposable absorbent article according to the preceding claim, wherein said open adhesive pattern is formed by lines of hot melt adhesive.

8. A breathable disposable absorbent article according to any one of the preceding claims, wherein said core has a caliper of less than 8 mm.

9. A breathable disposable absorbent article according to any one of the preceding claims, said dryness index is greater than 50, said Modified Sensory index is greater than 300, and said sensory index is greater than 550.

10. A breathable disposable absorbent article according to any one of the preceding claims wherein said article is a sanitary napkin.

**EP 1 040 799 A1**

European Patent Office

# EUROPEAN SEARCH REPORT

Application Number

EP 99 10 5191

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| D,X | EP 0 813 849 A (PROCTER & GAMBLE) 29 December 1997 (1997-12-29) * the whole document * | 1-4,7-10 | A61F13/15 |
| D,A | US 5 591 510 A (THOMAS PAUL E  ET AL) 7 January 1997 (1997-01-07) * claims; figures 19-22 * | 1,2,10 | |
| A | US 3 881 489 A (HARTWELL EDWARD WALLACE) 6 May 1975 (1975-05-06) * column 6, line 20 - line 55; claims; figures * | 1,2,5,10 | |
| D,A | EP 0 710 471 A (PROCTER & GAMBLE) 8 May 1996 (1996-05-08) * the whole document * | 1-5 | |

TECHNICAL FIELDS SEARCHED

A61F

The present search report has been drawn up for all claims

| Place of search | Date of completion of the search | Examiner |
|---|---|---|
| THE HAGUE | 9 September 1999 | Blas, V |

EPO FORM 1503 03.82 (P04C01)

**ANNEX TO THE EUROPEAN SEARCH REPORT
ON EUROPEAN PATENT APPLICATION NO.**                    EP 99 10 5191

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-1999

| Patent document cited in search report | | Publication date | Patent family member(s) | | Publication date |
|---|---|---|---|---|---|
| EP 0813849 | A | 29-12-1997 | AU | 3384797 A | 07-01-1998 |
| | | | AU | 3492897 A | 07-01-1998 |
| | | | CA | 2258484 A | 24-12-1997 |
| | | | CA | 2258527 A | 24-12-1997 |
| | | | CZ | 9804092 A | 12-05-1999 |
| | | | EP | 0813848 A | 29-12-1997 |
| | | | NO | 985855 A | 15-02-1999 |
| | | | NO | 985856 A | 10-02-1999 |
| | | | WO | 9748360 A | 24-12-1997 |
| | | | WO | 9748362 A | 24-12-1997 |
| | | | ZA | 9705341 A | 25-08-1998 |
| US 5591510 | A | 07-01-1997 | NONE | | |
| US 3881489 | A | 06-05-1975 | AT | 350015 B | 10-05-1979 |
| | | | AT | 673774 A | 15-10-1978 |
| | | | AU | 7234674 A | 19-02-1976 |
| | | | BE | 819013 A | 20-02-1975 |
| | | | CA | 1008655 A | 19-04-1977 |
| | | | CH | 577280 A | 15-07-1976 |
| | | | DE | 2439367 A | 27-02-1975 |
| | | | DK | 443874 A,B, | 14-04-1975 |
| | | | FR | 2241265 A | 21-03-1975 |
| | | | GB | 1471721 A | 27-04-1977 |
| | | | IE | 40440 B | 06-06-1979 |
| | | | IT | 1020028 B | 20-12-1977 |
| | | | JP | 50049041 A | 01-05-1975 |
| | | | LU | 70770 A | 11-06-1975 |
| | | | NL | 7411075 A,C | 24-02-1975 |
| | | | SE | 388342 B | 04-10-1976 |
| | | | SE | 7410532 A | 21-02-1975 |
| EP 0710471 | A | 08-05-1996 | AU | 708649 B | 12-08-1999 |
| | | | AU | 4005695 A | 31-05-1996 |
| | | | CA | 2203653 A | 17-05-1996 |
| | | | JP | 10509353 T | 14-09-1998 |
| | | | WO | 9614034 A | 17-05-1996 |
| | | | ZA | 9509259 A | 15-05-1996 |

EPO FORM P0459

For more details about this annex : see Official Journal of the European Patent Office, No. 12/82